# EUROPÄISCHE PATENTSCHRIFT

(11) **EP 2 089 718 B1**
(45) Veröffentlichungstag und Bekanntmachung des Hinweises auf die Patenterteilung: **31.08.2011**
(21) Anmeldenummer: 07846321.3
(22) Anmeldetag: 11.11.2007
(51) Int. Cl.: G01N 33/68

(54) **DIAGNOSE UND RISIKOSTRATIFIZIERUNG VON INFEKTIONEN UND CHRONISCHEN ERKRANKUNGEN DER ATEMWEGE UND LUNGE MITTELS PROVASOPRESSIN, INSBESONDERE COPEPTIN ODER NEUROPHYSIN II**
DIAGNOSIS AND RISK STRATIFICATION OF INFECTIONS AND CHRONIC DISEASES OF THE RESPIRATORY TRACT AND LUNGS BY MEANS OF PROVASOPRESSIN, PARTICULARLY COPEPTIN OR NEUROPHYSIN II
DIAGNOSTIC ET STRATIFICATION DES RISQUES D'INFECTIONS ET DE MALADIES CHRONIQUES DES VOIES RESPIRATOIRES ET DES POUMONS PAR PROVASOPRESSINE, NOTAMMENT COPEPTINE OU NEUROPHYSINE II

(30) Priorität: 12.11.2006 DE 102006053442
(43) Veröffentlichungstag der Anmeldung: 19.08.2009
(60) Teilanmeldung: 11170807.9
(73) Patentinhaber: B.R.A.H.M.S GmbH, 16761 Hennigsdorf (DE)
(72) Erfinder: BERGMANN, Andreas, 12351 Berlin (DE); MORGENTHALER, Nils, 13503 Berlin (DE); PAPASSOTIRIOU, Jana, 14163 Berlin (DE); STRUCK, Joachim, 13465 Berlin (DE); MÜLLER, Beat, 4059 Basel (CH)
(74) Vertreter: Simandi, Claus
(86) Internationale Anmeldenummer: PCT/DE2007/002037
(87) Internationale Veröffentlichungsnummer: WO 2008/058517

(56) Entgegenhaltungen:
- WO-A-2004/006860
- WO-A-2006/018315
- SELIGMAN RENATO ET AL: "Decreases in procalcitonin and C-reactive protein are strong predictors of survival in ventilator-associated pneumonia" CRITICAL CARE, BIOMED CENTRAL LTD., LONDON, GB, Bd. 10, Nr. 5, 6. September 2006 (2006-09-06), Seite R125, XP021020959 ISSN: 1364-8535
- STOLZ, D. ET AL: "Diagnostic value of signs, symptoms and laboratory values in lower respiratory tract infection" SWISS MED WKLY, Bd. 136, 2006, Seiten 434-440, XP002488253
- STOLZ DAIANA ET AL: "Copeptin, C-reactive protein, and procalcitonin as prognostic biomarkers in acute exacerbation of COPD." CHEST APR 2007, Bd. 131, Nr. 4, April 2007 (2007-04), Seiten 1058-1067, XP002488254 ISSN: 0012-3692

## Beschreibung

Die Erfindung betrifft ein Verfahren zur Diagnose und / oder Risikostratifizierung von Infektionen oder chronischen Erkrankungen der Atemwege und Lunge, insbesondere der tiefen Atemwegsinfektion (LRTI: Lower Respiratory Tract Infections) und COPD ("chronic obstructive pulmonary disease"), wobei eine Bestimmung des Provasopressins (proAVP) oder Fragmenten und Teilpeptiden davon, insbesondere Copeptin oder Neurophysin II erfolgt. Ferner betrifft die Erfindung hierzu geeignete Kombinationen von Biomarkern zur in-vitro Diagnostik.

Zwecks einer geeigneten Therapie, bedarf es einer frühen Diagnose und Differenzierung der Infektionen oder chronischen Erkrankungen der Atemwege und Lunge bereits in der Notaufnahme in Verbindung mit der Notwendigkeit klinische Entscheidungen zu treffen. Aufgrund unspezifischer Symptome (Atemnot) bei Infektionen oder chronischen Erkrankungen der Atemwege und Lunge ist sowohl die Differenzierung und Abgrenzung von anderen Erkrankungen als auch die Erkennung der konkreten Infektion oder chronischen Erkrankung der Atemwege und Lunge wesentlich.

Copeptin (auch: C-terminales proAVP) ist in WO 2006/018315 (BRAHMS AG) als Biomarker zur in-vitro Diagnose von Herzerkrankungen und zugehörigen pulmonaren Fehlfunktionen ("pulmonary disorders") beschrieben. Ein hierzu gehöriger Copeptin-Assay wird in Morgenthaler et al. (Nils G. Morgenthaler, Joachim Struck, Christine Alonso and Andreas Bergmann, Assay for the Measurement of Copeptin, a Stable Peptide Derived from the Precursor of Vasopressin Clinical Chemistry 52: 112-119, 2006) offenbart.

Im Stand der Technik ist die Procalcitonin (PCT)-Bestimmung beschrieben zwecks Untersuchung zur Abgrenzung einer bakteriellen Sepsis (Schwellenwert >0,5 ng/mL) von anderen Krankheitsursachen (EP0656121). PCT ist ebenfalls in Zusammenhang mit Pneumonien in der Literatur beschrieben, wobei sich die Untersuchungen hinsichtlich der Diagnose vor allem auf die Diskriminierung von unterschiedlichen Erregertypen bei bereits diagnostizierter Pneumonie beziehen (Prat C, Dominguez J, Andreo F, Blanco S, Pallares A, Cuchillo F, Ramil C, Ruiz-Manzano J, Ausina V, Procalcitonin and neopterin correlation with aetiology and severity of pneumonia, J Infect. 52(3) (2006): pp169-177 und Masia M, Gutierrez F, Shum C, Padilla S, Navarro JC, Flores E, Hernandez I, Masia M, Gutierrez F, Shum C, Padilla S, Navarro JC, Flores E, Hernandez I, Chest 128(4) (2005): pp 2223-2239, Boussekey N, Leroy O, Georges H, Devos P, d'Escrivan T, Guery B, Diagnostic and prognostic values of admission procalcitonin levels in community-acquired pneumonia in an intensive care unit. Infection 33(4) (2005): pp 257-63). In einer Arbeit von Zhou et al (Zhou CD et al Zhongguo Wei Zhong Bing Ji Jiu Yi Xue. 2006 Jun, 18(6),370-2) wird PCT als diagnostischer Marker für die frühe Diagnose einer Ventilator-assoziierten Pneumonie auf einer Intensivstation vorgestellt. Ebenfalls als prognostischer Marker ist das PCT bei Pneumonien beschrieben (Prat et al. 2006 (supra) und Masia M et al. 2005 (supra), Boussekey N et al. 2005 (supra), Christ-Crain M, Morgenthaler NG, Stolz D, Muller C, Bingisser R, Harbarth S, Tamm M, Struck J, Bergmann A, Muller B, Pro-adrenomedullin to predict severity and outcome in community-acquired pneumonia, Crit Care 10(3) (2006): pp R96). Es liegen zudem Untersuchungen vor, bei denen gezeigt wurde, dass mit Hilfe von PCT bei Patienten mit Verdacht auf Infektionen der unteren Atemwege (einschließlich Pneumonien) bei einer Schwellenwertkonzentration von >0,1 ng/mL bzw. >0,25 ng/mL klinisch relevante Infektionen (darunter ebenfalls bakterielle Pneumonien), die einer Antibiotikatherapie bedürfen, detektiert werden (Christ-Crain M, Stolz D, Bingisser R, Muller C, Miedinger D, Huber PR, Zimmerli W, Harbarth S, Tamm M, Muller B, Procalcitonin Guidance of Antibiotic Therapy in Community-acquired Pneumonia: A Randomized Trial, Am J Respir Crit Care Med 174(1) (2006), pp. 84-93 und Christ-Crain M, Jaccard-Stolz D, Bingisser R, Gencay MM, Huber PR, Tamm M, Muller B, Effect of procalcitonin-guided treatment on antibiotic use and outcome in lower respiratory tract infections: cluster-randomised, single-blinded intervention trial, Lancet 21; 363 (9409) (2004): pp 600-607, Stolz D, Christ-Crain M, Gencay MM, Bingisser R, Huber PR, Muller B, Tamm M, Diagnostic value of signs, symptoms and laboratory values in lower respiratory tract infection, Swiss Med Wkly 8: 136 (27-28) (2006): pp 434-440).

Eine Markerkombination von Copeptin und Procalcitonin ist jedoch nicht beschrieben.

Zu COPD sind bestimmte klinische Studien bekannt (Soler-Cataluna JJ, Martinez-Garcia MA, Roman Sanchez P, et al. Severe acute exacerbations and mortality in patients with chronic obstructive pulmonary disease. Thorax 2005; 60:925-931; Antonelli Incalzi R, Fuso L, De Rosa M, et al. Comorbidity contributes to predict mortality of patients with chronic obstructive pulmonary disease. Eur Respir J 1997; 10:2794-2800; Yohannes AM, Baldwin RC, Connolly MJ. Predictors of 1-year mortality in patients discharged from hospital following acute exacerbation of chronic obstructive pulmonary disease. Age Ageing 2005; 34:491-496; Almagro P, Barreiro B, Ochoa de Echaguen A, et al. Risk factors for hospital readmission in patients with chronic obstructive pulmonary disease. Respiration 2006; 73:311-317).

Nachteilig an bekannten Diagnoseverfahren unter Verwendung der bisher bekannten Markern ist jedoch, dass eine frühzeitige und vollständige Erfassung von Risikopatienten nicht gelingt und daher eine Risikostratifizierung nur ungenügend erfolgt. Eine der Erfindung zugrunde liegende Aufgabe besteht daher darin, ein Verfahren zur Risikostratifizierung zur Diagnose und / oder Risikostratifizierung von Infektionen oder chronischen Erkrankungen der Atemwege und Lunge zu entwickeln, das eine verbesserte Erfassung von Risikopatienten ermöglicht.
Ferner ist nachteilig, dass im Stand der Technik zumeist keine hinreichende Sensitivität und/oder Spezifität der Marker erreicht wird.
Eine weitere Aufgabe besteht daher darin, ein Verfahren zur Diagnose und / oder Risikostratifizierung von Infektionen oder chronischen Erkrankungen der Atemwege und Lunge bereitzustellen, wobei mindestens ein Marker oder eine Kombination von Marker eine hinreichende Sensitivität und Spezifität aufweist.

Daher ist es Aufgabe der vorliegenden Erfindung ein Verfahren zur Diagnose und / oder Risikostratifizierung von Infektionen oder chronischen Erkrankungen der Atemwege und Lunge bereitzustellen.

Die Aufgabe wird durch ein Verfahren zur Diagnose und / oder Risikostratifizierung von Infektionen oder chronischen Erkrankungen der Atemwege und Lunge gelöst, wobei eine Bestimmung des Provasopressins (proAVP) oder Fragmenten und Teilpeptiden davon, insbesondere Copeptin oder Neurophysin II erfolgt (nachstehend erfindungsgemäßes Verfahren).

Überraschender Weise weisen Provasopressins (proAVP) oder Fragmenten und Teilpeptiden davon, vorzugsweise Copeptin oder Neurophysin II eine hohe Sensitivität und Spezifität für die Diagnose von Infektionen oder chronischen Erkrankungen der Atemwege und Lunge auf.

Im Rahmen dieser Erfindung wird unter "Infektionen der Lunge und Atemwege" insbesondere solche Infektionen verstanden, die durch Bakterien, Viren, Pilze oder Parasiten verursacht werden, z.B. solche Indikationen wie tiefe Atemwegsinfektion (LRTI: Lower respiratory tract infections), Bronchitis, Pneumonie, Sarkoidose, Bronchiektasen, Nicht-kardiales Lungenödem.
Erfindungsgemäß bevorzugt sind zudem tiefe Atemwegsinfektion (LRTI: Lower respiratory tract infections), Bronchitis, putride Bronchitis, Pneumonie. Ganz besonders bevorzugt ist Pneumonie, insbesondere die ambulant erworbene Pneumonie (CAP: community associated pneumonie), tiefe Atemwegsinfektion (LRTI: Lower respiratory tract infections).

Im Rahmen dieser Erfindung wird unter Pneumonie eine akute oder chronische Erkrankung des Lungengewebes verstanden und dessen Infektion ist verursacht durch Bakterien, Viren oder Pilze, Parasiten, selten auch toxisch durch Inhalation giftiger Stoffe oder immunologisch. Für den Kliniker ist die Pneumonie eine Konstellation verschiedener Symptome (Fieber oder Hypothermie, Schüttelfrost, Husten, pleuritischer Thoraxschmerz, gesteigerte Sputumproduktion, erhöhte Atemfrequenz, Klopfschalldämpfung, Bronchialatmen, ohrnahe Rasselgeräusche, Pleurareiben) in Kombination mit mindestens einem auf dem Thorax-Röntgenbild erkennbaren Infiltrat (Harrisons Innere Medizin, herausgegeben von Manfred Dietel, Norbert Suttorp und Martin Zeitz, ABW Wissenschaftsverlag 2005

Im Rahmen dieser Erfindung wird unter "chronische Erkrankungen der Lunge und Atemwege" solche Indikationen verstanden, wie Interstitielle Lungenerkrankungen und Lungenfibrosen, Chronisch obstruktive Lungenerkrankungen (COPD) insbesondere COPD Infekt-Exazerbationen, Asthma bronchiale, insbesondere Infekt-Exazerbationen bei Asthma bronchiale, Bronchialkarzinom. Ganz besonders bevorzugt ist COPD, insbesondere COPD Infekt-Exazerbationen.

COPD bezeichnet erfindungsgemäß eine Gruppe von chronischen Krankheiten, die durch Husten, vermehrten Auswurf und Atemnot bei Belastung gekennzeichnet sind. In erster Linie sind die chronisch-obstruktive Bronchitis und das Lungenemphysem zu nennen. Beide Krankheitsbilder sind dadurch gekennzeichnet, dass vor allem die Ausatmung (Exspiration) behindert ist. Eine umgangssprachliche Bezeichnung für das Hauptsymptom der COPD ist zudem "Raucherhusten". Die Erfindung ist besonders vorteilhaft bei akuten Exazerbationen.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Infektionen oder chronischen Erkrankungen der Atemwege und Lunge mit dem Ziel eines möglichst günstigen Verlaufs zu ermöglichen. Eine erfindungsgemäße Risikostratifizierung erlaubt in Folge ein effektives Behandlungsverfahren, die bei Infektionen oder chronischen Erkrankungen der Atemwege und Lunge mit Arzneimitteln, insbesondere Antibiotika gegeben sind.

Daher betrifft die Erfindung ebenfalls die Identifizierung von Patienten mit erhöhtem Risiko oder / und einer ungünstigen Prognose von Infektionen oder chronischen Erkrankungen der Atemwege und Lunge, und zwar bei symptomatischen und / oder asymptomatischen Patienten, insbesondere Notfallpatienten.

Besonders vorteilhaft kann insbesondere in Fällen der Notfallund /oder Intensivmedizin mittels des erfindungsgemäßen Verfahren eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Eine solche klinische Entscheidung umfasst erfindungsgemäß ebenfalls die Hospitalisierung der Patienten.

Daher betrifft die Erfindung ebenfalls ein Verfahren zur Diagnose und / oder Risikostratifizierung von Infektionen oder chronischen Erkrankungen der Atemwege und Lunge zur Durchführung von klinischen Entscheidungen, wie weiterführende Behandlung und Therapie mittels Arzneimitteln, insbesondere Antibiotika, vorzugsweise in der zeitlich kritischen Intensivmedizin oder Notfallmedizin.

In einer weiteren bevorzugten Ausführungsform betrifft das erfindungsgemäße Verfahren daher die Therapiesteuerung von Infektionen oder chronischen Erkrankungen der Atemwege und Lunge.

In einer weiteren bevorzugten Ausführungsform des erfindungsgemäßen Verfahrens erfolgt die Diagnose und / oder Risikostratifizierung zur Prognose, zur differentialdiagnostischen Früherkennung und Erkennung, zur Beurteilung des Schweregrades und zur therapiebegleitenden Verlaufsbeurteilung.
In einer weiteren bevorzugten Ausführungsform betrifft die Erfindung ein Verfahren zur in-vitro Diagnose und / oder Risikostratifizierung von Infektionen oder chronischen Erkrankungen der Atemwege und Lunge, wobei eine Bestimmung des Marker Provasopressins (proAVP) oder Fragmenten und Teilpeptiden davon, insbesondere Copeptin oder Neurophysin II, an einem zu untersuchenden Patienten durchgeführt wird. Besonders bevorzugt ist jedoch Copeptin oder ein Fragment oder eine Teilsequenz davon. Insbesondere zeigt Copeptin zu Zwecken der in-vitro Diagnostik eine vorteilhafte hohe Stabilität im Serum und Plasma (Morgenthaler NG, Struck J, Alonso C, et al. Assay for the measurement of copeptin, a stable peptide derived from the precursor of vasopressin. Clin Chem 2006; 52:112-119).

Ferner betrifft die Erfindung ein Verfahren zur Diagnose und / oder Risikostratifizierung von Infektionen oder chronischen Erkrankungen der Atemwege und Lunge oder ein Verfahren zur in-vitro Diagnostik zur Früh- oder Differentialdiagnose oder Prognose von Infektionen oder chronischen Erkrankungen der Atemwege und Lunge nach einem der obigen Ausführungsformen, wobei nach Eintreten der Symptome ein cut-off (Schwellenwert) Bereich von 6-20 pmol/L der Marker Provasopressins (proAVP) oder Fragmenten und Teilpeptiden davon, insbesondere Copeptin oder Neurophysin II signifikant (spezifisch) für die Diagnose und / oder Risikostratifizierung ist. Weiterhin bevorzugt ist ein cut-off (Schwellenwert) von 6-10 pmol/L, insbesondere 9 pmol/L, vorzugsweise bis 2 Stunden nach Eintreten der Symptome.

Ferner betrifft die Erfindung ein Verfahren zur Diagnose und / oder Risikostratifizierung von Infektionen oder chronischen Erkrankungen der Atemwege und Lunge oder ein Verfahren zur in-vitro Diagnostik zur Früh- oder Differentialdiagnose oder Prognose von Infektionen oder chronischen Erkrankungen der Atemwege und Lunge nach einem der obigen Ausführungsformen, wobei nach Eintreten der Symptome ein cut-off (Schwellenwert) Bereich von 10-50 pmol/L der Marker Provasopressins (proAVP) oder Fragmenten und Teilpeptiden davon, insbesondere Copeptin oder Neurophysin II signifikant (spezifisch) für die Prognose und / oder Risikostratifizierung ist. Weiterhin bevorzugt ist ein cut-off (Schwellenwert) von 10-40 pmol/L.

Aus dieser Basis sind diese erfindungsgemäßen Verfahren vorteilhaft sensitiv.

In einer Ausführungsform des erfindungsgemäßen Verfahrens wird dem zu untersuchenden Patienten Körperflüssigkeit, insbesondere Blut entnommen, wahlweise Vollblut oder Serum oder erhältliches Plasma und die Diagnose erfolgt in *vitro*/*ex* vivo, d.h. außerhalb des menschlichen oder tierischen Körpers. Aufgrund der Bestimmung des Markers Provasopressins (proAVP) oder Fragmenten und Teilpeptiden davon, insbesondere Copeptin oder Neurophysin II wird eine hohe Sensitivität und Spezifität für Infektionen oder chronischen Erkrankungen der Atemwege und Lunge erzielt und anhand der vorhandenen Menge in mindestens einer Patientenprobe kann die Diagnose oder Risikostratifizierung erfolgen. Ganz besonders bevorzugt ist jedoch der Marker Copeptin (stabiles Fragment aus proAVP bzw. Preprovasopressin) oder ein Fragment oder eine Teilsequenz davon.

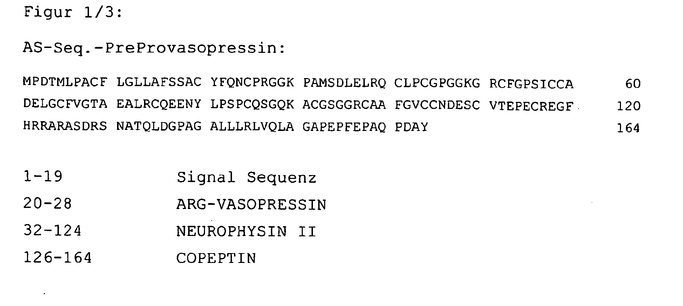

Im Rahmen dieser Erfindung wird unter "Provasopressin" ein humanes Protein oder Polypeptid verstanden, dass aus dem Preprovasopressin erhalten werden kann und im Rahmen des Preprovasopressins die Aminosäuren 29 - 164 umfasst (Siehe ebenfalls WO2006/018315 und Figur 1) und daraus erhältliche Fragmente oder Teilpeptide, insbesondere Copeptin (Fragment: AS 126-164 (39AS: SEQ: ASDRSNATQL DGPAGALLLR LVQLAGAPEP FEPAQPDAY) oder Neurophysin II (Fragment: AS 32-124 des Preprovasopressins (93AS: SEQ: AMSDLELRQC LPCGPGGKGR CFGPSICCAD ELGCFVGTAE ALRCQEENYL PSPCQSGQKA CGSGGRCAAF GVCCNDESCV TEPECREGFH RRA). Ferner können diese erfindungsgemäße Polypeptide posttranslationale Modifikationen aufweisen, wie Glykolisierung, Lip(o)idisierung oder Derivatisierungen.

In einer weiteren Ausführungsform kann die Bestimmung von Provasopressins (proAVP) oder Fragmenten und Teilpeptiden davon, insbesondere Copeptin oder Neurophysin II zusätzlich mit weiteren Markern erfolgen und zwar vorzugsweise solche, die bereits auf Infektionen oder chronischen Erkrankungen der Atemwege und Lunge hinweisen.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein.

In einer besonders bevorzugten Ausführungsform betrifft die Erfindung eine besonders vorteilhafte Kombination von Biomarker und zwar Provasopressins (proAVP) oder Fragmenten und Teilpeptiden davon, insbesondere Copeptin oder Neurophysin II mit Procalcitonin (1-116, 3-116) und / oder der Gruppe C-reaktivem Protein (CRP).

Daher betrifft die Erfindung ein Verfahren zur in-vitro Diagnose und / oder Risikostratifizierung von Infektionen oder chronischen Erkrankungen der Atemwege und Lunge, wobei eine Bestimmung des Marker Provasopressins (proAVP) oder Fragmenten und Teilpeptiden davon, insbesondere Copeptin oder Neurophysin II in Kombination mit Procalcitonin (1-116, 3-116) oder jeweils eine Teilsequenz davon, an einem zu untersuchenden Patienten durchgeführt wird. Besonders bevorzugt ist wiederum eine Kombination aus Neurophysin II, Copeptin und Procalcitonin, insbesondere Copeptin und Procalcitonin. Besonders vorteilhaft weisen diese genannten Biomarkerkombinationen Synergien auf, die zu einer verbesserten Spezifität und Sensitivität zur Diagnose des führen.

Im Rahmen dieser Erfindung wird unter "Procalcitonin" ein humanes Protein oder Polypeptid verstanden mit einer Aminosäuresequenz von 1-116 AS oder 2-116 AS (PCT 2-116) oder 3-116 AS (PCT 3-116), wie in der EP0656121, EP1121600 der Anmelderin sowie DE10027954A1 beschrieben. Ferner kann das erfindungsgemäße Procalcitonin postranslationale Modifikationen aufweisen, wie Glykolisierung, Lip(o)idisierung oder Derivatisierungen. Ferner umfasst sind ebenfalls Teilsequenzen oder Fragmente von Procalcitonin.

In einer weiteren Ausführungsform der Erfindung kann das erfindungsgemäße Verfahren im Rahmen einer in-vitro Diagnose mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen in einer diagnostischen Vorrichtung anhand eines Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung. In einer besonders bevorzugten Ausführungsform handelt es sich um einen Selbsttest oder um eine Vorrichtung, die in der Notfalldiagnostik geeignet ist.

Ferner betrifft die Erfindung die Verwendung von , Provasopressins (proAVP) oder Fragmenten und Teilpeptiden davon, insbesondere Copeptin oder Neurophysin II zur Verfahren zur in-vitro Diagnose und / oder Risikostratifizierung von Infektionen oder chronischen Erkrankungen der Atemwege und Lunge und/oder zur in-vitro Diagnostik zur Früh- oder Differentialdiagnose oder Prognose von Infektionen oder chronischen Erkrankungen der Atemwege und Lunge.

In einer besonderen Ausführungsform betrifft die Erfindung die Verwendung von Provasopressins (proAVP) oder Fragmenten und Teilpeptiden davon, insbesondere Copeptin oder Neurophysin II in Kombination mit Procalcitonin (1-116, 3-116) oder jeweils eine Teilsequenz davon, zur Diagnose und / oder Risikostratifizierung von Infektionen oder chronischen Erkrankungen der Atemwege und Lunge.

Die erfindungsgemäße Verwendung kann ggfs. mit mindestens einem weiteren geeigneten Marker erfolgen (so genannte "Panel" oder "Cluster").

Eine weitere Aufgabe ist die Verwendung einer entsprechenden diagnostischen Vorrichtung zur Durchführung der erfindungsgemäßen Verfahren.

Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung der erfindungsgemäßen Verfahren.

Die Erfindung betrifft zudem die Verwendung eines kits zur Diagnose und / oder Risikostratifizierung von Infektionen oder chronischen Erkrankungen der Atemwege und Lunge, enthaltend Nachweisreagenzien zur Bestimmung des Provasopressins (proAVP) oder Fragmenten und Teilpeptiden davon, insbesondere Copeptin oder Neurophysin II ggfs. oben genannten weiteren Marker. Solche Nachweisreagenzien umfassen z.B. Antikörper etc.

Nachfolgende Beispiele und Figuren dienen zur näheren Erläuterung der Erfindung, jedoch ohne die Erfindung auf diese Beispiele und Figuren zu beschränken.

### Beispiele und Figuren:

### Beispiel 1

Patienten, die sich mit dem Leitsymptom der Atemnot in der Notaufnahme eines Krankenhauses vorgestellt haben, wurde während der Eingangsuntersuchung eine Blutprobe entnommen. Zahl der Probanden: 167

### Probengewinnung, Biomarker-Analytik:

Blutabnahmen erfolgten mittels Standard-Serum-Monovetten. Nach einer Gerinnungszeit von 20-40 min erfolgte eine Zentrifugation für 15 min bei 2000 g die anschließende Serumtrennung erfolgte durch Dekantieren. Bis zur weiteren Verwendung wurden die Serumproben bei -20 Grad C gelagert.

Copeptin-Assay gemäß Morgenthaler et al (Morgenthaler NG, Struck J, Alonso C, et al. Assay for the measurement of copeptin, a stable peptide derived from the precursor of vasopressin. Clin Chem 2006; 52:112-119)
Nachweisgrenze; 1,7 pmol/L,

### COPD bei akuter Verschlimmerung (Ereignis):

Es konnte festgestellt werden, dass Copeptin, CRP und Procalcitonin nach 14 Tagen oder 6 Monaten signifikant erhöht sind.
Copeptin Werte von 167 Patienten (Alter (mittel) 70 Jahre) wurden untersucht. Die Patienten kamen auf die Notfallaufnahme mit akuter Exazerbation einer bestehenden COPD. Die Patienten wurden klinisch hinsichtlich ihrer Lungenfunktion bei Aufnahme, nach 14 Tagen und nach 6 Monaten untersucht. Tabelle 1 ergibt einen Überblick über die klinischen Parameter der Patienten bei Aufnahme auf die Notfallstation.

| Tabelle 1: Klinische Parameter bei Aufnahme von 167 Patienten mit akuter Exazerbation einer COPD | |
|---|---|
| Parameter | n = 167 |
| Geschlecht (M/F) (%) | 75/92 (44.9/55.1) |
| Alter in Jahren (von bis) | 70 (42-91) |
| Rauchen Packungen/Jahr | 45 (30-60) |
| Durchschnittl. Dauer COPD Monate (SD) | 127 (86) |
| Dauer der Exazerbation in Tage | 4 (3-7) |
| Husten (%) | 142 (85) |
| Vermehrte Sputum Produktion (%) | 113 (67.7) |
| Verfärbtes Sputum (%) | 95 (56.9) |
| Atemnot (%) | 155 (92.8) |
| Fieber (%) | 68 (40.7) |
| Co-Morbidität (%) | |
| Kardiopathie | 76 (45.5) |
| Tumor | 24 (14.4) |
| Diabetes Mellitus | 19 (11.4) |
| | |
| Schwere der COPD - GOLD Stage (%) | |
| I | |
| II | 10 (6.0) |
| III | 35 (21.0) |
| IV | 74 (44.3) |
| | 48 (28.7) |
| FEV1 in Liter (SD) | 0.892 (0.397) |
| FEV1% erwartet (%) | 39.9 (16.9) |
| PaO₂ mmHg | 62.9 (15.7) |
| PaCO₂ mmHg | 43.8 (11.0) |
| Leukocyten x 109/l (SD) | 11.27 (4.7) |

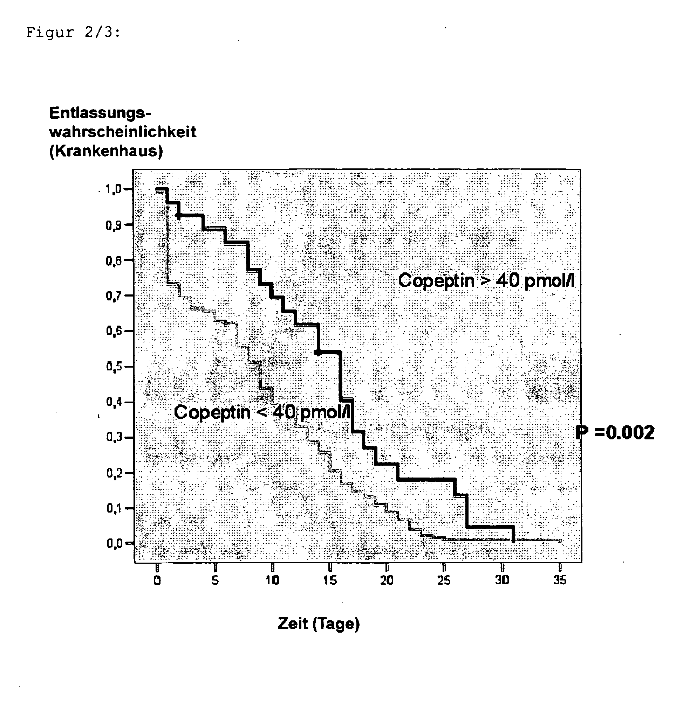

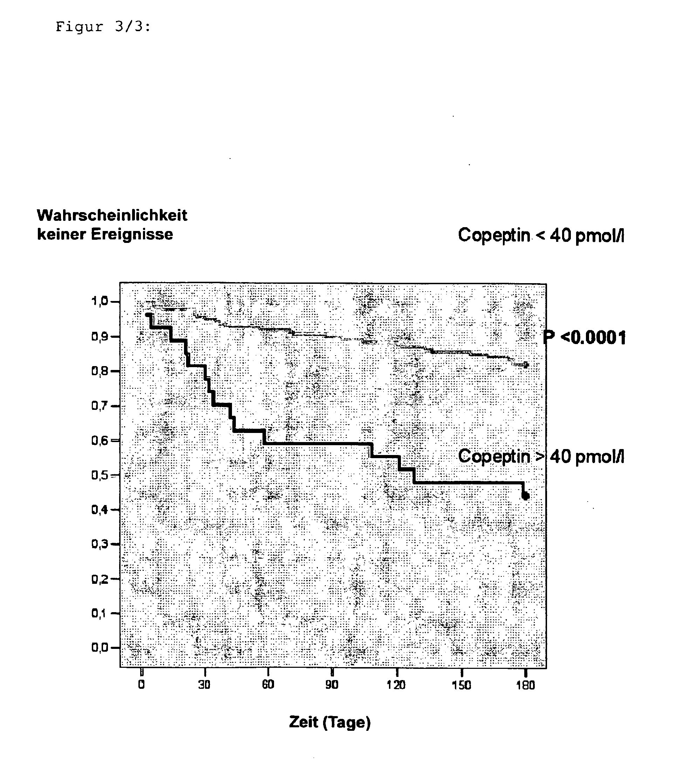

In einer Multi-Varianten Analyse (p=0.006, Cox-Regressions Analyse) war der gemessene Copeptin Wert ein Prediktor für einen schlechten klinischen Verlauf, unabhängig vom Alter, bestehender Co-Morbidität, einer Hypoxämie und einer eingeschränkten Lungenfunktion. Eine erfolgreiche Langzeittherapie konnte nur bei 44 Prozent der Patienten mit Copeptin Werten ≥ 40 pmol/l bei Aufnahme erreicht werden. Im Gegensatz dazu war dies bei 82 Prozent der Patienten mit Copeptin Werten < 40 pmol/l erreichbar (p<0.0001).
Daraus ergibt sich, dass Copeptin bei Patienten mit COPD (und anderen Lungenerkrankungen) ein Prognosemarker für einen ungünstigen klinischen Verlauf darstellt. Copeptin könnte die Patienten früh identifizieren, die eine besonders intensive Therapie ihrer bestehenden Lungenerkrankung benötigen. Tabelle 2 sowie Figuren 2 und 3 erläutern den Sachverhalt im Detail.

| Tabelle 2: Vergleich des klinischen Ergebnis von Patienten mit Copeptin levels < 40 pmol/l und ≥ 40 pmol/l bei Aufnahme | | | |
|---|---|---|---|
| | Copeptin < 40 pmol/l n = 140 | Copeptin ≥ 40 pmol/l n = 27 | p-Wert |
| Hospitalisierungsdauer <24 hours % (n) | 26.4% (37) | 3.7% (1) | 0.010 |
| Dauer der Hospitalisierung Tage (IQR) | 8.5 (1-15) | 14 (8-18) | 0.002 |
| Notwendigkeit für Intensivstation % (n) | 6.4% (9) | 25.9% (7) | 0.002 |
| Dauer Intensivstation Tage (IQR) | 2 (1.5-4.5) | 5 (3-5) | 0.097 |
| Hospitalisierungsrate innerhalb von 6 Monaten | 0.17 ± 0.4 | 0.55 ± 0.74 | 0.005 |
| Durchschnittliche Zeit bis zum klinischen Versagen | 163 ± 45 | 111 ± 73 | <<0.001 |
| Langfristiges klinisches Versagen % (n) | 17.9% (25) | 55.6% (15) | <<0.001 |
| Tod während Hospitalisierung % (n) | 1.4% (2) | 11.1% (3) | 0.007 |
| Tod innerhalb von 6 Monaten % (n) | 5% (7) | 25.9% (7) | <<0.001 |

### Figuren:

Figur 1 zeigt die Aminosäuresequenz von preproVasopressin.

Figur 2 zeigt den Zusammenhang zwischen Liegezeiten im Krankenhaus von Patienten mit CAP/Pneumonie und dem gemessenen Copeptin Plasmawert bei Aufnahme. Dies ist gezeigt als Entlassungswahrscheinlichkeit über die Zeit ("probability of hospital discharge"). Patienten mit Copeptin Werten < 40 pmol/l bei Aufnahme hatten einen signifikant kürzeren stationären Aufenthalt im Vergleich zu Patienten mit Copeptin Werten > 40 pmol/l.
Anzahl Patienten an den Angegebenen Tagen (d):
Copeptin < 40 pmol/l:
   n =140 (0 d) 88 (5 d) 54 (10 d) 28 (15 d) 12 (20 d)1 (25 d) 1 (30 d) 0 (35 d)
Copeptin ≥ 40 pmol/l:
   n = 27 (0 d) 23 (5 d) 18 (10 d) 12 (15 d) 5 (20 d)4 (25 d) 1 (30 d) 0 (35 d)

Figur 3 zeigt den Zusammenhang zwischen der Häufigkeit von Komplikationen (Tod, erneute statiönäre Aufnahme nach Entlassung) und dem Copeptin Plasmawert bei Aufnahme. Dies ist gezeigt als Ereignis freie Zeit (freedom of event). Ereignis (event) ist definiert als Tod oder erneute stationäre Aufnahme nach initialer Entlassung. Patienten mit Copeptin Werten < 40 pmol/l bei Aufnahme hatten signifikant seltener dieses Ereignis als Patienten mit Copeptin Werten > 40 pmol/l.
Anzahl Patienten an den Angegebenen Tagen (d):
Copeptin. < 40 pmol/l:
   n = 140 (0 d) 133 (30 d) 129 (60 d) 126 (90 d) 123 (120 d) 120 (150 d) 115 (180 d)
Copeptin > 40 pmol/l:
   n = 27 (0 d) 21 (30 d) 16 (60 d) 16 (90 d) 15 (120 d) 13 (150 d) 12 (180 d)

## Patentansprüche

1. In vitro Verfahren zur Diagnose und / oder Risikostratifizierung von Infektionen oder chronischen Erkrankungen der Atemwege und Lunge ausgewählt aus der Gruppe: Chronisch obstruktive Lungenerkrankungen (COPD), COPD Infekt-Exazerbationen, Asthma bronchiale, Infekt-Exazerbationen bei Asthma bronchiale, Bronchitis, putride Bronchitis, Bronchiektasien, wobei eine Bestimmung des Provasopressins (proAVP) oder Copetin oder Neurophysin II in einer körperflüssigkeitsprobe erfolgt.

2. Verfahren zur Diagnose und / oder Risikostratifizierung von Infektionen oder chronischen Erkrankungen der Atemwege und Lunge nach Anspruch 1, zur Identifizierung von Patienten mit erhöhtem Risiko oder / und einer ungünstigen Prognose von Infektionen oder chronischen Erkrankungen der Atemwege und Lunge.

3. Verfahren zur Diagnose und / oder Risikostratifizierung von Infektionen oder chronischen Erkrankungen der Atemwege und Lunge nach Anspruch 1 oder 2, wobei der Patient ein symptomatischer und / oder asymptomatischer Patient, insbesondere ein Notfallpatient ist.

4. Verfahren zur Diagnose und / oder Risikostratifizierung von Infektionen oder chronischen Erkrankungen der Atemwege und Lunge nach einem der Ansprüche 1 bis 3 zur Durchführung von klinischen Entscheidungen, insbesondere weiterführende Behandlung und Therapie mittels Arzneimitteln, insbesondere Antibiotika, insbesondere in der Intensivmedizin oder Notfallmedizin und zur Hospitalisierung des Patienten.

5. Verfahren zur Diagnose und / oder Risikostratifizierung von Infektionen oder chronischen Erkrankungen der Atemwege und Lunge nach einem der Ansprüche 1 bis 4, wobei eine Bestimmung des Marker Provasopressins (proAVP) oder Copeptin oder Neurophysin II in Kombination mit Procalcitonin und / oder der Gruppe C-reaktivem Protein (CRP) oder jeweils eine Teilsequenz davon, an einem zu untersuchenden Patienten durchgeführt wird.

6. Verfahren zur Diagnose und / oder Risikostratifizierung von Infektionen oder chronischen Erkrankungen der Atemwege und Lunge nach einem der Ansprüche 1 bis 5, **dadurch gekennzeichnet, dass** die Diagnose zur Prophylaxe, zur Prognose, zur differentialdiagnostischen Früherkennung und Erkennung, zur Beurteilung des Schweregrades und zur therapiebegleitenden Verlaufsbeurteilung erfolgt.

7. Verfahren nach einem der vorherigen Ansprüche,
**dadurch gekennzeichnet,**
**dass** der cut-off (Schwellenwert) zur Diagnose 6-20 pmol/L, insbesondere 6-10 pmol/L oder der cut-off (Schwellenwert) zur Prognose 10-50 pmol/L, insbesondere 10-40 pmol/L der Marker Provasopressins (proAVP) oder Copeptin oder Neurophysin II signifikant (spezifisch) ist.

8. Verfahren nach einem der vorherigen Ansprüche,
**dadurch gekennzeichnet,**
**dass** zusätzlich eine Bestimmung mindestens eines weiteren Marker ausgewählt aus der Gruppe inflammatorischer Marker durchgeführt wird.

9. Verfahren nach einem der vorherigen Ansprüche, **dadurch gekennzeichnet, dass** der inflammatorische Marker aus mindestens einem Marker aus der Cytokinen, wie etwa TNF-alpha, Interleukinen, wie etwa IL-6 und Adhäsionsmolekülen, wie VCAM oder ICAM ausgewählt ist.

10. Verfahren nach einem der vorherigen Ansprüche,
**dadurch gekennzeichnet,**
**dass** parallele oder simultane Bestimmungen der Marker durchgeführt werden.

11. Verfahren nach einem der vorherigen Ansprüche,
**dadurch gekennzeichnet,**
**dass** die Bestimmungen an mindestens einer Patientenprobe durchgeführt werden.

12. Verfahren nach einem der vorherigen Ansprüche,
**dadurch gekennzeichnet,**
**dass** die Bestimmungen an einem Analyseautomaten, insbesondere mittels einem Kryptor, durchgeführt werden.

13. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

14. Verfahren nach einem der vorherigen Ansprüche, **dadurch gekennzeichnet, dass** die Risikostratifizierung zur Prognose, zur differentialdiagnostischen Früherkennung und Erkennung, zur Beurteilung des Schweregrades und zur therapiebegleitenden Verlaufsbeurteilung des akuten Koronarsyndroms erfolgt.

15. Verwendung in vitro von Provasopressins (proAVP) oder Copeptin oder Neurophysin II und ggfs. mindestens ein weiterer Marker zur Diagnose und / oder Risikostratifizierung von Infektionen oder chronischen Erkrankungen der Atemwege und Lunge ausgewählt aus der Gruppe: Chronisch obstruktive Lungenerkrankungen (COPD), COPD Infekt-Exazerbationen, Asthma bronchiale, Infekt-Exazerbationen bei Asthma bronchiale, Bronchitis, putride Bronchitis, Bronchiektasien.

16. Verwendung von Provasopressins (proAVP) oder Copeptin oder Neurophysin II in Kombination mit Procalcitonin (1-116, 3-116) und / oder der Gruppe C-reaktivem Protein (CRP) und ggfs. mindestens ein weiterer Marker zur Diagnose und / oder Risikostratifizierung in vitro von Infektionen oder chronischen Erkrankungen der Atemwege und Lunge ausgewählt aus der Gruppe: Chronisch obstruktive Lungenerkrankungen (COPD), COPD Infekt-Exazerbationen, Asthma bronchiale, Infekt-Exazerbationen bei Asthma bronchiale, Bronchitis, putride Bronchitis, Bronchiektasien.

17. Verwendung eines Kits zur Diagnose und / oder Risikostratifizierung in vitro von Infektionen oder chronischen Erkrankungen der Atemwege und Lunge ausgewählt aus der Gruppe: Chronisch obstruktive Lungenerkrankungen (COPD), COPD Infekt-Exazerbationen, Asthma bronchiale, Infekt-Exazerbationen bei Asthma bronchiale, , Bronchitis, putride Bronchitis, Bronchiektasien, enthaltend Antikörper zur Bestimmung von Provasopressins (proAVP) oder Copeptin oder Neurophysin II und ggfs. weitere Marker nach einem der Ansprüche 8 bis 9 und Hilfsmittel.

## Claims

1. In-vitro method for diagnosis and/or risk stratification of infections or chronic diseases of the airways and lungs selected from the group of chronic obstructive pulmonary disease (COPD), COPD infection exacerbations, bronchial asthma, infection exacerbations with bronchial asthma, bronchitis, putrid bronchitis, bronchiectases, wherein a determination of provasopressin (proAVP) or copeptin or neurophysin II takes place in a sample of bodily fluid.

2. Method for diagnosis and/or risk stratification of infections or chronic diseases of the airways and lungs according to claim 1, for identifying patients having an increased risk and/or a disadvantageous prognosis of infections or chronic diseases of the airways and lungs.

3. Method for diagnosis and/or risk stratification of infections or chronic diseases of the airways and lungs according to claim 1 or 2, where the patient is a symptomatic and/or asymptomatic patient, particularly an emergency patient.

4. Method for diagnosis and/or risk stratification of infections or chronic diseases of the airways and lungs according to one of claims 1 to 3, for carrying out clinical decisions, particularly further treatment and therapy by means of medications, particularly antibiotics, particularly in intensive-care medicine or emergency medicine, and for hospitalization of the patient.

5. Method for diagnosis and/or risk stratification of infections or chronic diseases of the airways and lungs according to one of claims 1 to 4, where a determination of the marker provasopressin (proAVP) or fragments and partial peptides thereof, or copeptin or neurophysin II, is carried out, in combination with procalcitonin and/or the group of C-reactive protein (CRP), or a partial sequence thereof, in each instance, in a patient to be examined.

6. Method for diagnosis and/or risk stratification of infections or chronic diseases of the airways and lungs according to one of claims 1 to 5, **characterized in that** the diagnosis takes place for prophylaxis, for prognosis, for early recognition and recognition by means of a differential diagnosis, for an assessment of the degree of severity, and for an assessment of the progression over the course of therapy.

7. Method according to one of the preceding claims, **characterized in that** the cut-off (threshold value) for diagnosis, 6-20 pmol/L, particularly 6-10 pmol/L, or the cut-off (threshold value) for prognosis, 10-50 pmol/L, particularly 10-40 pmol/L, of the marker provasopressin (proAVP) or fragments and partial peptides thereof, or copeptin or neurophysin II, is significant (specific).

8. Method according to one of the preceding claims, **characterized in that** in addition, a determination of at least one other marker selected from the group of inflammatory markers is carried out.

9. Method according to one of the preceding claims, **characterized in that** the inflammatory marker is selected from at least one marker of the cytokines, such as TNF-alpha, for example, interleukins, such as IL-6, for example, and adhesion molecules, such as VCAM or ICAM.

10. Method according to one of the preceding claims, **characterized in that** parallel or simultaneous determinations of the markers are carried out.

11. Method according to one of the preceding claims, **characterized in that** the determinations are carried out using at least one patient sample.

12. Method according to one of the preceding claims, **characterized in that** the determinations are carried out using an automated analysis device, particularly by means of a Kryptor.

13. Method according to one of the preceding claims, **characterized in that** the determinations are carried out by means of a rapid test, particularly with single-parameter or multi-parameter determinations.

14. Method according to one of the preceding claims, **characterized in that** the risk stratification takes place for prognosis, for early recognition and recognition by means of a differential diagnosis, for an assessment of the degree of severity, and for an assessment of the progression over the course of therapy, of acute coronary syndrome.

15. In-vitro use of provasopressin (proAVP) or copeptin or neurophysin II and where appropriate at least one additional marker for diagnosis and/or risk stratification of infections or chronic diseases of the airways and lungs selected from the group of chronic obstructive pulmonary disease (COPD), COPD infection exacerbations, bronchial asthma, infection exacerbations with bronchial asthma, bronchitis, putrid bronchitis, bronchiectases.

16. Use of provasopressin (proAVP) or copeptin or neurophysin II in combination with procalcitonin (1-116, 3-116) and/or the group of C-reactive protein (CRP), and where appropriate at least one additional marker for in-vitro diagnosis and/or risk stratification of infections or chronic diseases of the airways and lungs selected from the group of chronic obstructive pulmonary disease (COPD), COPD infection exacerbations, bronchial asthma, infection exacerbations with bronchial asthma, bronchitis, putrid bronchitis, bronchiectases.

17. Use of a kit for in-vitro diagnosis and/or risk stratification of infections or chronic diseases of the airways and lungs selected from the group of chronic obstructive pulmonary disease (COPD), COPD infection exacerbations, bronchial asthma, infection exacerbations with bronchial asthma, bronchitis, putrid bronchitis, bronchiectases, containing antibodies for determining provasopressin (proAVP) or copeptin or neurophysin II, and where appropriate other markers according to one of claims 8 to 9, and ancillary substances.

## Revendications

1. Procédé in vitro pour le diagnostic et/ou la stratification des risques d'infections ou de maladies chroniques des voies aériennes et des poumons, choisies dans le groupe de la maladie pulmonaire obstructive chronique (COPD), des exacerbations d'infection avec COPD, de l'asthme bronchique, d'exacerbations d'infection avec asthme bronchique, de la bronchite, de la bronchite putride, des bronchiectasies, dans lesquelles une détermination de la provasopressine (proAVP) ou de la copeptine ou de la neurophysine II a lieu dans un échantillon de fluide corporel.

2. Procédé de diagnostic et/ou de stratification des risques d'infections ou de maladies chroniques des voies aériennes et des poumons, selon la revendication 1, pour identifier des patients ayant un risque accru et/ou un pronostic désavantageux d'infections ou de maladies chroniques des voies aériennes et des poumons.

3. Procédé de diagnostic et/ou de stratification des risques d'infections ou de maladies chroniques des voies aériennes et des poumons, selon l'une des revendications 1 ou 2, où le patient est un patient symptomatique et/ou asymptomatique, en particulier un patient en situation d'urgence.

4. Procédé de diagnostic et/ou de stratification des risques d'infections ou de maladies chroniques des voies aériennes et des poumons, selon l'une des revendications 1 à 3, pour exécuter des décisions cliniques, en particulier une poursuite de traitement et une thérapie au moyen de médications, en particulier d'antibiotiques, en particulier en médecine de soins intensifs ou en médecine d'urgence, et pour l'hospitalisation du patient.

5. Procédé de diagnostic et/ou de stratification des risques d'infections ou de maladies chroniques des voies aériennes et des poumons, selon l'une des revendications 1 à 4, où une détermination du marqueur provasopressine (pro-AVP) ou fragments et peptides partiels de celle-ci, ou copeptine ou neurophysine II, est effectuée, en combinaison avec la procalcitonine et/ou le groupe de la protéine C-réactive (CRP), ou une séquence partielle de ceux-ci, dans chaque cas, dans un patient devant être examiné.

6. Procédé de diagnostic et/ou de stratification des risques d'infections ou de maladies chroniques des voies aériennes et des poumons, selon l'une des revendications 1 à 5, **caractérisé par le fait que** le diagnostic a lieu pour une prophylaxie, pour un pronostic, pour une reconnaissance précoce et une reconnaissance au moyen d'un diagnostic différentiel, pour une évaluation du degré de gravité et pour une évaluation de la progression au cours de la thérapie.

7. Procédé selon l'une des revendications précédentes, **caractérisé par le fait que** la coupure (valeur de seuil) pour le diagnostic, 6-20 pmol/L, en particulier 6-10 pmol/L, ou la coupure (valeur de seuil) pour le pronostic, 10-50 pmol/L, en particulier 10-40 pmol/L, du marqueur provasopressine (proAVP) ou fragments et peptides partiels de celle-ci, ou copeptine ou neurophysine II, est significative (spécifique).

8. Procédé selon l'une des revendications précédentes, **caractérisé par le fait qu'**en outre, une détermination d'au moins un autre marqueur choisi dans le groupe de marqueurs inflammatoires est effectuée.

9. Procédé selon l'une des revendications précédentes, **caractérisé par le fait que** le marqueur inflammatoire est choisi parmi au moins un marqueur des cytokines, tel que le TNF-alpha, par exemple des interleukines, telles que IL-6, par exemple, et des molécules d'adhésion, telles que VCAM ou ICAM.

10. Procédé selon l'une des revendications précédentes, **caractérisé par le fait que** des déterminations parallèles ou simultanées des marqueurs sont effectuées.

11. Procédé selon l'une des revendications précédentes, **caractérisé par le fait que** les déterminations sont effectuées à l'aide d'au moins un échantillon de patient.

12. Procédé selon l'une des revendications précédentes, **caractérisé par le fait que** les déterminations sont effectuées à l'aide d'un dispositif d'analyse automatisé, en particulier au moyen d'un Kryptor.

13. Procédé selon l'une des revendications précédentes, **caractérisé par** le fait les déterminations sont effectuées au moyen d'un test rapide, en particulier avec des déterminations à un seul paramètre ou à plusieurs paramètres.

14. Procédé selon l'une des revendications précédentes, **caractérisé par** le fait la stratification des risques a lieu pour un pronostic, pour une reconnaissance précoce et une reconnaissance au moyen d'un diagnostic différentiel, pour une évaluation du degré de gravité et pour une évaluation de la progression au cours de la thérapie, du syndrome coronarien aigu.

15. Utilisation in vitro de vasopressine (proAVP) ou de copeptine ou de neurophysine II et si approprié d'au moins un marqueur supplémentaire pour le diagnostic et/ou la stratification des risques d'infections ou de maladies chroniques des voies aériennes et des poumons, choisies dans le groupe de la maladie pulmonaire obstructive chronique (COPD), des exacerbations d'infection avec COPD, de l'asthme bronchique, des exacerbations d'infection avec asthme bronchique, de la bronchite, de la bronchite putride, des bronchiectasies.

16. Utilisation de provasopressine (proAVP) ou de copeptine ou de neurophysine II en combinaison avec la procalcitonine (1-116, 3-116) et/ou le groupe de la protéine C-réactive (CRP), et si approprié d'au moins un marqueur supplémentaire pour le diagnostic et/ou la stratification des risques in vitro d'infections ou de maladies chroniques des voies aériennes et des poumons, choisies dans le groupe de la maladie pulmonaire obstructive chronique (COPD), des exacerbations d'infection avec COPD, de l'asthme bronchique, des exacerbations d'infection avec asthme bronchique, de la bronchite, de la bronchite putride, des bronchiectasies.

17. Utilisation d'un coffret pour le diagnostic et/ou la stratification des risques in vitro d'infections ou de maladies chroniques des voies aériennes et des poumons, choisies dans le groupe de la maladie pulmonaire obstructive chronique (COPD), des exacerbations d'infection avec COPD, de l'asthme bronchique, des exacerbations d'infection avec asthme bronchique, de la bronchite, de la bronchite putride, des bronchiectasies, contenant des anticorps pour déterminer la provasopressine (proAVP) ou la copeptine ou la neurophysine II, et, si approprié, d'autres marqueurs selon l'une des revendications 8 à 9, et des substances auxiliaires.
